# EUROPEAN PATENT SPECIFICATION

(11) **EP 1 991 598 B1**
(45) Date of publication and mention of the grant of the patent: **21.04.2010**
(21) Application number: 07712235.6
(22) Date of filing: 19.02.2007
(51) Int. Cl.: C08G 18/48, B01J 23/02, C07C 41/03, C07H 15/08, C08G 65/26

(54) **PROCESS OF FORMING A POLYOL**
VERFAHREN ZUR BILDUNG EINES POLYOLS
PROCÉDÉ DE FORMATION D'UN POLYOL

(30) Priority: 27.02.2006 US 276370
(43) Date of publication of application: 19.11.2008
(73) Proprietor: BASF SE, 67056 Ludwigshafen (DE)
(72) Inventor: DEXHEIMER, Edward Michael, Surprise, AZ 85387 (US); HUANG, Mao-Yao, Riverview, Mi 48192 (US); PLEGUE, Thomas, Charlotte, Nc 28273 (US)
(86) International application number: PCT/EP2007/051543
(87) International publication number: WO 2007/096317

(56) References cited:
- EP-A- 0 049 358
- DE-A1- 10 322 784
- GB-A- 876 469

## Description

### FIELD OF THE INVENTION

The subject invention generally relates to a process of forming a polyol and a polyurethane article formed from the polyol. More specifically, the subject invention relates to a process of forming the polyol in the presence of an alkaline earth metal hydroxide and dimethylethanolamine

### DESCRIPTION OF THE RELATED ART

Various processes of forming polyols are known in the art. One process includes reacting sucrose and glycerin, together having an average functionality of less than 7, with an alkylene oxide. The sucrose, glycerin, and the alkylene oxide are typically reacted in the presence of catalytic amounts of potassium hydroxide and/or an amine to form the polyol. This reaction is an alkoxylation reaction and forms chains of alkylene oxide units on the hydroxyl groups of the sucrose and/or glycerin.

In the presence of the potassium hydroxide, either alone or with the amine, the alkoxylation reaction proceeds. However, the alkoxylation reaction adds additional and disproportionate numbers of alkylene oxide units to a pre-existing chain of alkylene oxide units that has been formed on the hydroxyl groups of the sucrose and/or glycerin. This leads to uneven chain lengths and un-reacted sucrose, which is a contaminant in the polyol. Additionally, use of potassium hydroxide leaves residues in the polyol which negatively affect foaming> properties. As a result, the potassium must be removed, thereby slowing manufacturing, decreasing product yield, and increasing production cost, which are not suitable for industrial practice.

In the presence of the amine alone, the alkoxylation reaction does not add additional and disproportionate numbers of alkylene oxide units. Yet, catalytic effectiveness of the amine is diminished as polymerization takes place, i.e., the number of moles of alkylene oxide per mole of the hydroxyl groups on the sucrose and/or glycerin increases. Specifically, the amine is not catalytically effective when the number of moles increase beyond two or when a hydroxyl number of the polyol falls below 350 mg KOH/g.

Attempts have been made to overcome issues associated with using potassium hydroxide and/or amines. However, these attempts are not cost effective and do not produce the polyol in high yield. One attempt includes a step-wise reaction of sucrose and glycerin with an alkylene oxide. Specifically, the sucrose and the glycerin are reacted with the alkylene oxide in the presence of the amine until the chain length is about one mole of the alkylene oxide per one mole of the hydroxyl groups of the sucrose and/or glycerin. At that point, the reaction is stopped and the potassium hydroxide is added to form the polyol. However, this step-wise reaction decreases production speed in that the manufacturing process must be stopped to add the potassium hydroxide. Additionally, this step-wise reaction decreases yield and increases costs making the step-wise reaction not desirable for commercial use.

It is also known in the art to form foams and surfactants through the use of a polyol and an isocyanate, in the presence of an alkaline earth metal hydroxide and an amine. However, in these situations, the alkaline earth metal hydroxide and the amine are not used to catalyze alkoxylation of an initiator composition to form the polyol and therefore do not function in an equivalent way when used to form the foam and/or surfactants.

Accordingly, there remains an opportunity to form a polyol having consistent chain length with increased speed and in high yield, while reducing costs and maximizing efficiency. There also remains an opportunity to form a polyurethane article from the polyol.

### SUMMARY OF THE INVENTION AND ADVANTAGES

The present invention provides a process of forming a polyol. The process includes the step of providing an alkylene oxide and the step of providing an initiator composition having an average functionality of at least four. The process also includes the step of providing an Alkaline earth metal hydroxide and dimethylethanolamine. The process further includes the step of reacting the initiator composition and the alkylene oxide in the presence of the alkaline earth metal hydroxide and the amine to form the polyol.

The combination of the alkaline earth metal hydroxide and the dimethylethanolamine allow for a smooth transition from amine catalysis to alkaline earth metal hydroxide catalysis to form the polyol. This eliminates a need to interrupt the process. This also allows the polyol to have consistent chain length and be formed with increased speed and in high yield, while reducing costs and maximizing efficiency.

### BRIEF DESCRIPTION OF THE SEVERAL VIEWS OF THE DRAWINGS

Other advantages of the present invention will be readily appreciated, as the same becomes better understood by reference to the following detailed description when considered in connection with the accompanying drawings wherein:

Figure 1 is a line graph illustrating an experimental process profile of polyol formation through alkoxylation of an initiator composition in the presence of dimethylethanolamine and strontium hydroxide 8-hydrate, and percentage of propylene oxide addition, pressure and temperature over time;

Figure 2 is a line graph illustrating a first control process profile of polyol formation through alkoxylation of an initiator composition in the presence of dimethylcyclohexylamine, and percentage of propylene oxide addition, pressure and temperature over time; and

Figure 3 is a line graph illustrating a second control process profile of polyol formation through alkoxylation of an initiator composition in the presence of strontium hydroxide 8-hydrate, and percentage of propylene oxide addition, pressure and temperature over time.

### DETAILED DESCRIPTION OF THE INVENTION

A process of forming a polyol is disclosed. The process includes the step of providing an alkylene oxide and the step of providing an initiator composition having an average functionality of at least four. The process also includes the step of providing an alkaline earth metal hydroxide and dimethylethanolamine. The step of providing the alkaline earth metal hydroxide and the amine may be further defined as providing a combination of the alkaline earth metal hydroxide and the amine. However, the alkaline earth metal hydroxide and the amine may be provided separately. If provided separately, the process preferably includes the step of providing the amine before providing the alkaline earth metal hydroxide. Alternatively, the process may include the step of providing the alkaline earth metal hydroxide before providing the amine.

The process further includes the step of reacting the initiator composition and the alkylene oxide in the presence of the alkaline earth metal hydroxide and the amine to form the polyol. The polyol, the alkaline earth metal hydroxide, and the amine will each be described in greater detail below. Additionally, the polyol formed from the process is also disclosed. Further, a polyurethane article including the reaction product of an isocyanate and the polyol formed from the process of the present invention is also disclosed. The polyurethane article and the process arc also described in greater detail below.

The initiator composition used to form the polyol has an average functionality of at least four. Preferably, the initiator composition has an average functionality of at least six. In one embodiment, the initiator composition includes sucrose, sorbitol, or a combination thereof. Preferably, the initiator composition includes sucrose, commercially available from Michigan Sugar Company of Bay City, MI, under the trade name of Big Chief Granulated Sugar. However, it is also contemplated that the initiator composition may include a non-reducing sugar having at least six hydroxyl groups, other than sucrose. It is also contemplated that combinations of the initiator composition may be utilized.

The initiator composition may include a blend of two or more initiators. In one embodiment, the initiator composition includes a first initiator selected from the group of sucrose, sorbitol, and combinations thereof and a second initiator selected from the group of glycerin, propylene glycol, dipropylene glycol, ethylene glycol, diethylene glycol, and combinations thereof. In another embodiment, the initiator composition includes sucrose and glycerin. If the initiator composition includes the second initiator, the second initiator may be any known in the art and may include low molecular weight di- and/or poly-functional alcohols and amines. Preferably, the second initiator has a functionality of from 2 to 6 and more preferably of from 3 to 5. Most preferably, the second initiator has a functionality of 3. In another embodiment, the second initiator includes glycerin, commercially available from Proctor and Gamble under the trade name of Superol^{®}. Other suitable second initiators may also be used and may include trimethylol-alkanes such as 1,1,1-trimethylolpropane. It is contemplated that combinations of the second initiator may also be utilized.

The first initiator and/or the second initiator may be present in the initiator composition in any amount. In one embodiment, the first initiator is preferably present in an amount of at least 30 parts by weight per 100 parts by weight of the initiator composition. Also, the second initiator is preferably present in an amount of less than 40, more preferably of less than 30, and most preferably of less than 20, parts by weight per 100 parts by weight of the initiator composition. In one embodiment, the second initiator is present in an amount such that the amount of the first initiator and the amount of the second initiator are together equivalent to the total amount of the initiator composition.

The initiator composition reacts with the alkylene oxide in the presence of the alkaline earth metal hydroxide and the dimethylethanolamine, to form the polyol. The alkylene oxide may be any alkylene oxide known in the art and is preferably selected from the group of ethylene oxide, propylene oxide, butylene oxide, amylene oxide, and combinations thereof. More preferably the alkylene oxide is selected from the group of ethylene oxide, propylene oxide, and combinations thereof. Most preferably, the alkylene oxide includes propylene oxide. The initiator composition may react with the alkylene oxide in any amount dependent on the goals of one skilled in the art to form the polyol having specific hydroxyl numbers. This reaction of the initiator composition and the alkylene oxide is a ring opening alkoxylation reaction and forms chains of alkylene oxide units on the hydroxyl groups of the first initiator and/or second initiator, thereby forming the polyol. Preferably, this reaction forms the polyol having approximately equal length chains of the alkylene oxide units, thereby allowing the polyol to have consistent physical and chemical properties.

Referring now to the alkaline earth metal hydroxide first introduced above, the polyol is formed in the presence of the alkaline earth metal hydroxide. Alkaline earth metals are located in Group 2 of the Periodic Table, as opposed to the alkali metals located in Group 1 of the Periodic Table. The present invention docs not include alkali metals located in Group 1. The alkaline earth metal hydroxide is preferably selected from the group of calcium hydroxide, strontium hydroxide, barium hydroxide, and combinations thereof. Most preferably, the alkaline earth metal hydroxide includes strontium hydroxide. The alkaline earth metal hydroxide may also include hydrates. Examples of suitable hydrates include, but are not limited to, 1 through 9 hydrates, and combinations thereof. A particularly suitable hydrate includes 8-hydrate (octahydrate). In one embodiment, the alkaline earth metal hydroxide includes strontium hydroxide 8-hydrate commercially available from Noah Technologies Corporation of San Antonio, TX. Preferably, the alkaline earth metal hydroxide is present with the initiator composition, the alkylene oxide, and the amine in an amount of from 0.01 to 1, more preferably of from 0.1 to 0.75, and most preferably of from 0.2 to 0.5, parts by weight per 100 parts by weight of the polyol.

In addition to the alkaline earth metal hydroxide, the polyol is also formed, and the initiator composition and the alkylene oxide are reacted, in the presence of the amine. The amine is dimethylethanolamine, commercially available from Atofina Chemicals, Inc. of Philadelphia, PA. Preferably, the amine is present with the initiator composition, the alkylene oxide, and the alkaline earth metal hydroxide in an amount of from 0.01 to 5, more preferably of from 0.1 to 2, and most preferably of from 0.5 to 1.5, parts by weight per 100 parts by weight of the polyol.

Without intending to be bound by any particular theory, it is believed that the alkaline earth metal hydroxide catalytically supplements the amine. This catalyzes the reaction of the initiator composition and the alkylene oxide without interfering with an even distribution of alkylene oxide units on the hydroxyl groups of the first initiator and/or second initiator of the initiator composition, through alkoxylation. When an average chain length of the polyol is less than one (i.e., when there is less than one mole of alkylene oxide per one mole of hydroxyl groups of the initiator composition), it is believed that the amine is the dominant catalyst. When the chain length of the polyol is about 1.5, it is believed that a catalytic effect of the alkaline earth metal hydroxide accelerates. The combination of the alkaline earth metal hydroxide and the amine allows for a smooth transition from amine catalysis to alkaline earth metal hydroxide catalysis of the reaction of the initiator composition and the alkylene oxide to form the polyol. The polyol may be formed in any amount of time. However, the polyol is preferably formed in less than 15 hours, more preferably in less than 12 hours, and most preferably in less than 10 hours.

As first introduced above, the polyol includes the reaction product of the initiator composition having an average functionality of at least four and an alkylene oxide, reacted in the presence of the alkaline earth metal hydroxide and the amine. The polyol preferably has a hydroxyl number of less than or equal to 500, more preferably of from 200 to 470, and most preferably of from 280 to 370, mg KOH/g. Further, the polyol preferably has an equivalent weight of less than or equal to 300 Daltons. The terminology "equivalent weight" is a portion of the weight average molecular weight (M_{w}) of the polyol divided by a functionality of the polyol.

In one embodiment, the polyol that is formed includes an internal block formed from the alkylene oxide. In another embodiment, the polyol includes two internal blocks formed from the alkylene oxide. In yet another embodiment, the polyol includes three or more blocks formed from the alkylene oxide. In one embodiment, the polyol includes internal blocks including at least one ethylene oxide unit and at least one propylene oxide unit arranged in a heteric formation, i.e., random blocks formed from propylene oxide and/or ethylene oxide. At a minimum, it is preferred that the blocks have 50 parts by weight of propylene oxide per 100 parts by weight of the polyol. Also, the heteric formation preferably includes less than or equal to 3 repeating propylene oxide units.

After formation, the polyol may be used to form the polyurethane article. The polyurethane article may be a rigid or elastic foam or may be an elastomer. Preferably, the polyurethane article is a rigid foam. If a rigid foam, the polyurethane article may be used in a wide variety of industries including, but not limited to, in insulation and in building supplies. Before the polyol is used to form the polyurethane article, the alkaline earth metal hydroxide and the amine may be substantially removed from the polyol. However, this is not required.

The polyurethane article includes the reaction product of the isocyanate and the polyol, as first introduced above. However, before the polyol is reacted with the isocyanate, one or more additives may be added to the polyol and/or the isocyanate. The additive may include, but is not limited to, air releasing agents, wetting agents, surface modifiers, waxes, inert inorganic fillers, molecular sieves, reactive inorganic fillers, chopped glass, processing additives, surface-active agents, adhesion promoters, anti-oxidants, dyes, pigments, ultraviolet light stabilizers, thixotropic agents, anti-aging agents, lubricants, adhesion promoters, coupling agents, solvents, rheology promoters, surfactants, cross-linking agents, blowing agents, blowing reaction modifiers, catalysts including blowing, polymerization, and gelling catalysts, compatibilizers, chain extenders, anti-foaming agents, chain terminators, and combinations thereof. If included, the additive may be present in the polyol and/or the isocyanate in any amount. Additionally, a second polyol, different from the polyol, may also be added to the polyol before the polyurethane article is formed.

The isocyanate that reacts with the polyol may be any isocyanate known in the art and may include, but is not limited to, isocyanates, polyisocyanates, biurets of isocyanates and polyisocyanates, isocyanurates of isocyanates and polyisocyanates, and combinations thereof. In one embodiment of the present invention, the isocyanate component includes an n-functional isocyanate. In this embodiment, n is a number preferably from 2 to 5, more preferably from 2 to 4, and most preferably from 3 to 4. It is to be understood that n may be an integer or may have intermediate values from 2 to 5. The isocyanate may be selected from the group of aromatic isocyanates, aliphatic isocyanates, and combinations thereof. In one embodiment, the isocyanate component includes an aliphatic isocyanate. If the isocyanate component includes an aliphatic isocyanate, the isocyanate component may also include a modified multivalent aliphatic isocyanate, i.e., a product which is obtained through chemical reactions of aliphatic diisocyanates and/or aliphatic polyisocyanates. Examples include, but are not limited to, ureas, biurets, allophanates, carbodiimides, uretonimines, isocyanurates, urethane groups, dimers, trimers, and combinations thereof. The isocyanate component may also include, but is not limited to, modified diisocyanates employed individually or in reaction products with polyoxyalkyleneglycols, diethylene glycols, dipropylene glycols, polyoxyethylene glycols, polyoxypropylene glycols, polyoxypropylenepolyoxethylene glycols, polyesterols, polycaprolactones, and combinations thereof.

Alternatively, the isocyanate may include an aromatic isocyanate. If the isocyanates includes an aromatic isocyanate, the aromatic isocyanate may correspond to the formula R'(NCO), wherein R' is a polyvalent organic radical which is aromatic and z is an integer that corresponds to the valence of R'. Preferably, z is at least two. If the isocyanate includes the aromatic isocyanate, the isocyanate may include, but is not limited to, the tetramethylxylylene diisocyanate (TMXDI), 1,4-diisocyanatobenzene, 1,3-diisocyanato-o-xylene, 1,3-diisocyanato-p-xylene, 1,3-diisocyanato-m-xylene, 2,4-diisocyanato-1-chlorobenzene, 2,4-diisocyanato-1-nitrobenzene, 2,5-diisocyanato-1-nitrobenzene, m-phenylene diisocyanate, p-phenylene diisocyanate, 2,4-toluene diisocyanate, 2,6-toluene diisocyanate, mixtures of 2,4- and 2,6-toluene diisocyanate, 1,5-naphthalene diisocyanate, 1-methoxy-2,4-phenylene diisocyanate, 4,4'-diphenylmethane diisocyanate, 2,4'-diphenylmethane diisocyanate, 4,4'-biphenylene diisocyanate, 3,3'-dimethyl-4,4'-diphenylmethane diisocyanate, 3,3'-dimethyldiphenylmethane-4,4'-diisocyanate, triisocyanates such as 4,4',4"-triphenylmethane triisocyanate polymethylene polyphenylene polyisocyanate and 2,4,6-toluene triisocyanate, tetraisocyanates such as 4,4'-dimethyl-2,2'-5,5'-diphenylmethane tetraisocyanate, toluene diisocyanate, 2,2'-diphenylmethane diisocyanate, 2,4'-diphenylmethane diisocyanate, 4,4'-diphenylmethane diisocyanate, polymethylene polyphenylene polyisocyanate, corresponding isomeric mixtures thereof, and combinations thereof. Alternatively, the aromatic isocyanate may include a triisocyanate product of m-TMXDI and 1,1,1-trimethylolpropane, a reaction product of toluene diisocyanate and 1,1,1-trimethyolpropane, and combinations thereof. The isocyanate may have any % NCO content and any viscosity. The isocyanate may also react with the polyol in any amount to form the polyurethane article.

### EXAMPLES

A Polyol 1 is formed according to the process of the present invention in the presence of the Alkaline Earth Metal Hydroxide and a First Amine. Two comparative polyols, Comparative Polyols 1 and 2, are also formed but not according to the process of the present invention. The Comparative Polyol I is formed from reacting the Initiator Composition and the Alkylene Oxide in the presence of a Second Amine and not the Alkaline Earth Metal Hydroxide. The Comparative Polyol 2 is formed from reacting the Initiator Composition and the Alkylene Oxide in the presence of the Alkaline Earth Metal Hydroxide and not the First Amine or the Second Amine. Specific amounts of the Initiator Composition, the Alkylene Oxide, the Alkaline Earth Metal Hydroxide, the First Amine, and the Second Amine, are set forth in Table 1, below. The times of reaction, formulation OH numbers, and experimental OH numbers of the Polyol 1 and the Comparative Polyols 1 and 2, are also set forth below, in Table 1. All components are in grams for Polyol 1 and Comparative Polyol I and are in kilograms for Comparative Polyol 2, unless otherwise indicated.

**TABLE 1**

| **Initiator Composition** | **Polyol 1** | **Comparative Polyol 1** | **Comparative Polyol 2** |
|---|---|---|---|
| Glycerin | 1300 | 1420 | 78.88 |
| Sucrose | 2400 | 2160 | 125.28 |
| **Alkaline Earth Metal Hydroxide** | 22.5 | 0 | 1.31 |
| **First Amine** | 75 | 0 | 0 |
| **Second Amine** | 0 | 75 | 0 |
| **Alkylene Oxide** | 11,900 | 11,457 | 664.51 |
| Formulation OH Number (mg KOH/g) | 359.7 | 360.9 | 359.8 |
| Experimental OH Number (mg *KOH/g) | 355.6 | 386.9 | 3.60.9 |
| Time of Alkylene Oxide Addition (hrs) | 7.78 | 10.28 | 17.67 |
| Total Time of Reaction (hrs) | 9.5 | 12.9 | 18.66 |

Glycerin is commercially available from Procter & Gamble Company under the trade name of Superol^{®}.

Sucrose is commercially available from Michigan Sugar Company of Bay City, MI, under the trade name of Big Chief Granulated Sugar.

Alkaline Earth Metal Hydroxide is strontium hydroxide 8-hydrate, which is commercially available from Noah Technologies Corporation of San Antonio, TX.

First Amine is dimethylethanolamine, which is commercially available from Atofina Chemicals, Inc. of Philadelphia, PA.

Second Amine is dimethylcyclohexylamine, which is commercially available from Air Products and Chemicals, Inc. of Allentown, PA under the trade name of Polycat^{®} 8.

Alkylene Oxide is propylene oxide, which is commercially available from Huntsman Base Chemicals.

The Formulation OH Number is the OH number (mg KOH/g) of the Polyol 1 and the Comparative Polyols 1 and 2 that are calculated to result from the reaction of the Initiator Composition and the Alkylene Oxide.

The Experimental OH Number is the OH number (mg KOH/g) of the Polyol 1 and the Comparative Polyols 1 and 2 that actually result from the reaction of the Initiator Composition and the Alkylene Oxide.

The Time of Alkylene Oxide Addition is a time needed to add the Alkylene Oxide to a reactor vessel while maintaining a pressure of the reactor vessel at or below 6.2 bar [90 psig].

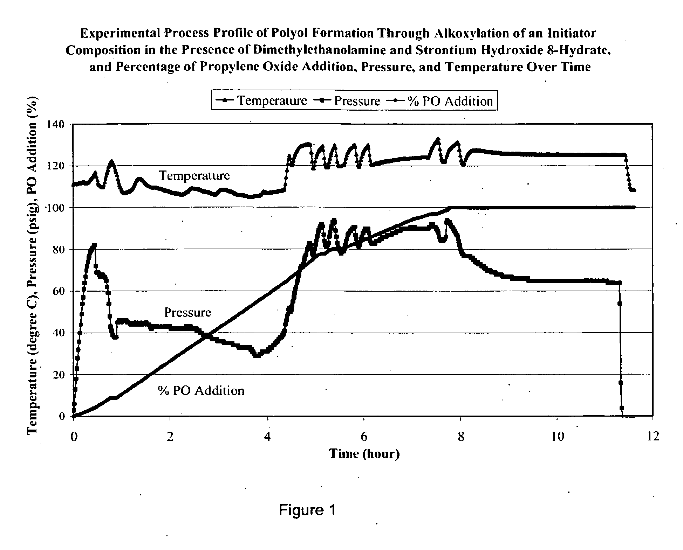

The Total Time of Reaction is a time needed to add the Alkylene Oxide to the reactor vessel and to complete alkoxylation of the Initiator Composition, after completion of the Alkylene Oxide addition, as measured in the reactor vessel for pressure, temperature, and time, and is shown in Figures 1 through 3. Specifically, the alkoxylation reaction is essentially complete when the pressure and temperature both achieve a steady state.

In Figure 1, alkoxylation of the Initiator Composition and formation of Polyol I is shown as being catalyzed by both the First Amine and the Alkaline Earth Metal Hydroxide. Without intending to be bound by any particular theory, it is believed that, as shown in Figure 1, between approximately 1 and 4 hours, the pressure is declining as a result of catalysis from the First Amine. It is also believed that, as shown in Figure 1, between approximately 4.3 and 8 hours, the catalysis from the First Amine is ending and catalysis from the Alkaline Earth Metal Hydroxide is increasing. Overall, at approximately 9 hours, the alkoxylation reaction is essentially complete when both the temperature and pressure achieve a steady state, thereby forming the Polyol 1 having an Experimental Hydroxyl Number of approximately 355.6 mg KOH/g, which is approximately equal to the Formulation Hydroxyl Number.

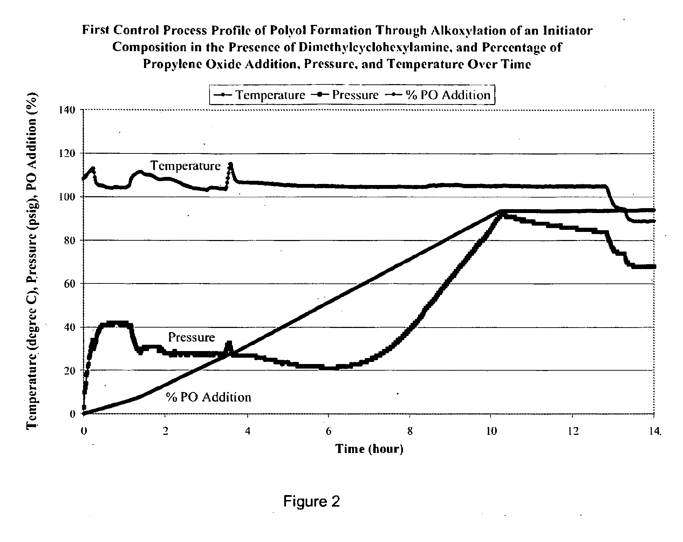

As shown in Figure 2 and in Table 1, the Comparative Polyol 1 forming in the presence of the Second Amine, dimethylcyclohexylamine, does not have an Experimental Hydroxyl Number that is approximately equal to the Formulation Hydroxyl Number. Figure 2 shows that forming the Comparative Polyol 1 is stopped at approximately 10.28 hours and 93.6% of the Alkylene Oxide amount added due to pressure considerations. Reaction of the added Alkylene Oxide is complete after the temperature and pressure become steady at approximately 12.9 hours. The remaining 6.4% of the Alkylene Oxide is subsequently added between 16.8 and 17.3 hours after an additional amount of the Second Amine, dimethylcyclohexylamine, is added to the reactor vessel. The last 6.4% of the Alkylene Oxide is later recovered in a vacuum-stripping operation and appears not to react to form the desired Comparative Polyol 1. Without intending to be limited by any particular theory, it is believed that the catalysis by the Second Amine includes a kinetic limitation on the formation of the Comparative Polyol 1. The kinetic limitation is believed to prevent the Comparative Polyol I from having an Experimental Hydroxyl Number that is approximately equal to the Formulation Hydroxyl Number.

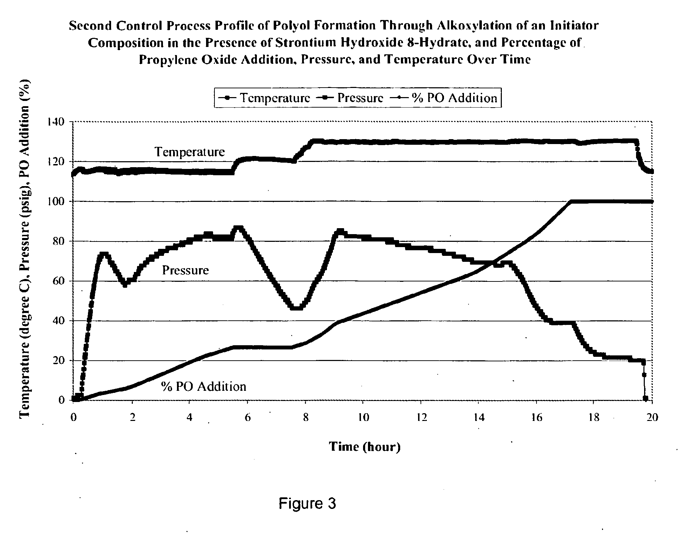

As is shown in Figure 3 and in Table 1, the Comparative Polyol 2 forming in the presence of strontium hydroxide has an Experimental Hydroxyl Number that is approximately equal to the Formulation Hydroxyl Number. However, Figure 3 also shows that forming the Comparative Polyol 2 is stopped at approximately 5.5 hours due to pressure considerations. After pressure decreases, the reaction begins again to form the Comparative Polyol 2. Rapid pressure increases are not desirable for industrial implementation.

In formation of the Polyol 1, the Time of Alkylene Oxide Addition is less than the corresponding times in the formation of the Comparative Polyols 1 and 2. Additionally, the total time of reaction for the formation of the Polyol 1 is also less than the corresponding total time of reaction for the formation of the Comparative Polyols 1 and 2.

After formation, the Polyol 1 exhibits an Experimental OH Number that is within approximately 1 percent of the Formulation OH Number and a reaction time that is less than 10 hours. Conversely, the Comparative Polyols 1 and 2 exhibit Experimental OH Numbers that are within 7.5 percent and 0.5 percent of the Formulation OH Numbers, respectively. However, the reactions times for the Comparative Polyols 1 and 2 are 12.9 hours and 18.66 hours, respectively, exhibiting inefficiency.

## Claims

1. A process of forming a polyol comprising the steps of:
providing an alkylene oxide;
providing an initiator composition having an average functionality of at least four;
providing an alkaline earth metal hydroxide and dimethylethanolamine; and
reacting the initiator composition and the alkylene oxide in the presence of the alkaline earth metal hydroxide and the dimethylethanolamine to form the polyol.

2. A process as set forth in claim 1 wherein the initiator composition comprises sucrose, sorbitol, or a combination thereof.

3. A process as set forth in claim 2 wherein the initiator composition comprises sucrose.

4. A process as set forth in claim 1 wherein the initiator composition comprises a blend of two or more initiators.

5. A process as set forth in claim 4 wherein the initiator composition comprises sucrose and glycerin.

6. A process as set forth in claim 4 wherein the initiator composition comprises a first initiator selected from the group of sucrose, sorbitol, and combinations thereof and a second initiator selected from the group of glycerin, propylene glycol, dipropylene glycol, ethylene glycol, diethylene glycol, and combinations thereof.

7. A process as set forth in claim 6 wherein the first initiator is present in an amount of at least 30 parts by weight per 100 parts by weight of the initiator composition.

8. A process as set forth in claim 1 wherein the alkaline earth metal hydroxide is selected from the group of calcium hydroxide, strontium hydroxide, barium hydroxide, and combinations thereof.

9. A process as set forth in claim 1 wherein the alkaline earth metal hydroxide comprises strontium hydroxide.

10. A process as set forth in claim 1 wherein the alkaline earth metal hydroxide is present in an amount from 0.1 to 0.75 parts by weight per 100 parts by weight of polyol.

11. A process as set forth in claim 1 wherein the amine is present in an amount from 0.1 to 2 parts by weight per 100 parts by weight of the polyol.

12. A process as set forth in claim 1 wherein the alkylene oxide is selected from the group of ethylene oxide, propylene oxide, butylene oxide, amylene oxide, and combinations thereof.

13. A process as set forth in claim 1 wherein the alkylene oxide comprises propylene oxide.

14. A process as set forth in claim 1 wherein the polyol has a hydroxyl number of from 200 to 470 mg KOH/g and an equivalent weight of less than or equal to 300 Daltons.

15. A process as set forth in claim 1 wherein the polyol comprises internal blocks comprising at least one ethylene oxide unit and at least one propylene oxide unit arranged in a heteric formation.

16. A process as set forth in claim 15 wherein the heteric formation comprises less than or equal to 3 repeating propylene oxide units.

17. A process as set forth in claim 1 wherein the polyol is formed in less than 12 hours.

18. A process as set forth in claim 1 wherein the step of providing the alkaline earth metal hydroxide and the amine is further defined as providing a combination of the alkaline earth metal hydroxide and the dimethylethanolamine.

19. A process as set forth in claim 1 wherein the step of providing the alkaline earth metal hydroxide and the amine further comprises the step of providing the dimethylethanolamine before providing the alkaline earth metal hydroxide.

## Patentansprüche

1. Verfahren zur Bildung eines Polyols, bei dem man:
ein Alkylenoxid bereitstellt;
eine Starterzusammensetzung mit einer durchschnittlichen Funktionalität von mindestens vier bereitstellt;
ein Erdalkalimetallhydroxid und Dimethylethanolamin bereitstellt und
die Starterzusammensetzung und das Alkylenoxid in Gegenwart des Erdalkalimetallhydroxids und des Dimethylethanolamins zu dem Polyol umsetzt.

2. Verfahren nach Anspruch 1, bei dem die Starterzusammensetzung Saccharose, Sorbitol oder eine Kombination davon umfaßt.

3. Verfahren nach Anspruch 2, bei dem die Starterzusammensetzung Saccharose umfaßt.

4. Verfahren nach Anspruch 1, bei dem die Starterzusammensetzung eine Mischung von zwei oder mehr Startern umfaßt.

5. Verfahren nach Anspruch 4, bei dem die Starterzusammensetzung Saccharose und Glycerin umfaßt.

6. Verfahren nach Anspruch 4, bei dem die Starterzusammensetzung einen ersten Starter aus der Gruppe Saccharose, Sorbitol oder eine Kombination davon und einen zweiten Starter aus der Gruppe Glycerin, Propylenglykol, Dipropylenglykol, Ethylenglykol, Diethylenglykol und Kombinationen davon umfaßt.

7. Verfahren nach Anspruch 6, bei dem der erste Starter in einer Menge von mindestens 30 Gewichtsteilen pro 100 Gewichtsteile der Starterzusammensetzung vorliegt.

8. Verfahren nach Anspruch 1, bei dem man das Erdalkalimetallhydroxid aus der Gruppe Calciumhydroxid, Strontiumhydroxid, Bariumhydroxid und Kombinationen davon auswählt.

9. Verfahren nach Anspruch 1, bei dem das Erdalkalimetallhydroxid Strontiumhydroxid umfaßt.

10. Verfahren nach Anspruch 1, bei dem das Erdalkalimetallhydroxid in einer Menge von 0,1 bis 0,75 Gewichtsteilen pro 100 Gewichtsteile Polyol vorliegt.

11. Verfahren nach Anspruch 1, bei dem das Amin in einer Menge von 0,1 bis 2 Gewichtsteilen pro 100 Gewichtsteile des Polyols vorliegt.

12. Verfahren nach Anspruch 1, bei dem man das Alkylenoxid aus der Gruppe Ethylenoxid, Propylenoxid, Butylenoxid, Amylenoxid und Kombinationen davon auswählt.

13. Verfahren nach Anspruch 1, bei dem das Alkylenoxid Propylenoxid umfaßt.

14. Verfahren nach Anspruch 1, bei dem das Polyol eine Hydroxylzahl von 200 bis 470 mg KOH/g und ein Äquivalentgewicht kleiner gleich 300 Dalton aufweist.

15. Verfahren nach Anspruch 1, bei dem das Polyol innenständige Blöcke, die mindestens eine Ethylenoxid-Einheit und mindestens eine Propylenoxid-Einheit, die in statistischer Formation angeordnet sind, umfassen, umfaßt.

16. Verfahren nach Anspruch 15, bei dem die statistische Formation kleiner gleich 3 Propylenoxid-Wiederholungseinheiten umfaßt.

17. Verfahren nach Anspruch 1, bei dem das Polyol in weniger als 12 Stunden gebildet wird.

18. Verfahren nach Anspruch 1, bei dem der Schritt der Bereitstellung des Erdalkalimetallhydroxids und des Amins ferner als Bereitstellung einer Kombination des Erdalkalimetallhydroxids und des Dimethylethanolamins definiert ist.

19. Verfahren nach Anspruch 1, bei dem der Schritt der Bereitstellung des Erdalkalimetallhydroxids und des Amins ferner den Schritt der Bereitstellung des Dimethylethanolamins vor der Bereitstellung des Erdalkalimetallhydroxids umfaßt.

## Revendications

1. Procédé de formation d'un polyol, comprenant les étapes de :
mise à disposition d'un oxyde d'alkylène ;
mise à disposition d'une composition d'initiateurs ayant une fonctionnalité moyenne d'au moins quatre ;
mise à disposition d'un hydroxyde de métal alcalino-terreux et de diméthyléthanolamine ; et
mise en réaction de la composition d'initiateurs et de l'oxyde d'alkylène en présence de l'hydroxyde de métal alcalino-terreux et de la diméthyléthanolamine pour former le polyol.

2. Procédé selon la revendication 1, dans lequel la composition d'initiateurs comprend du sucrose, du sorbitol ou une de leurs combinaisons.

3. Procédé selon la revendication 2, dans lequel la composition d'initiateurs comprend du sucrose.

4. Procédé selon la revendication 1, dans lequel la composition d'initiateurs comprend un mélange de deux initiateurs ou plus.

5. Procédé selon la revendication 4, dans lequel la composition d'initiateurs comprend du sucrose et de la glycérine.

6. Procédé selon la revendication 4, dans lequel la composition d'initiateurs comprend un premier initiateur choisi dans le groupe du sucrose, du sorbitol et leurs combinaisons, et un second initiateur choisi dans le groupe de la glycérine, du propylène glycol, du dipropylène glycol, de l'éthylène glycol, du diéthylène glycol et leurs combinaisons.

7. Procédé selon la revendication 6, dans lequel le premier initiateur est présent en une quantité d'au moins 30 parties en poids pour 100 parties en poids de la composition d'initiateurs.

8. Procédé selon la revendication 1, dans lequel l'hydroxyde de métal alcalino-terreux est choisi dans le groupe de l'hydroxyde de calcium, l'hydroxyde de strontium, l'hydroxyde de baryum et leurs combinaisons.

9. Procédé selon la revendication 1, dans lequel l'hydroxyde de métal alcalino-terreux comprend de l'hydroxyde de strontium.

10. Procédé selon la revendication 1, dans lequel l'hydroxyde de métal alcalino-terreux est présent en une quantité de 0,1 à 0,75 parties en poids pour 100 parties en poids de polyol.

11. Procédé selon la revendication 1, dans lequel l'amine est présente en une quantité de 0,1 à 2 parties en poids pour 100 parties en poids du polyol.

12. Procédé selon la revendication 1, dans lequel l'oxyde d'alkylène est choisi dans le groupe de l'oxyde d'éthylène, l'oxyde de propylène, l'oxyde de butylène, l'oxyde d'amylène et leurs combinaisons.

13. Procédé selon la revendication 1, dans lequel l'oxyde d'alkylène comprend de l'oxyde de propylène.

14. Procédé selon la revendication 1, dans lequel le polyol a un indice hydroxyle de 200 à 470 mg KOH/g et un poids équivalent inférieur ou égal à 300 Daltons.

15. Procédé selon la revendication 1, dans lequel le polyol comprend des séquences internes qui comprennent au moins une unité oxyde d'éthylène et au moins une unité oxyde de propylène disposées selon une formation hétérique.

16. Procédé selon la revendication 15, dans lequel la formation hétérique comprend un nombre inférieur ou égal à 3 unités de répétition oxyde de propylène.

17. Procédé selon la revendication 1, dans lequel le polyol est formé en moins de 12 heures.

18. Procédé selon la revendication 1, dans lequel l'étape de mise à disposition de l'hydroxyde de métal alcalino-terreux et de l'amine est également définie comme la mise à disposition d'une combinaison de l'hydroxyde de métal alcalino-terreux et de la diméthyléthanolamine.

19. Procédé selon la revendication 1, dans lequel l'étape de mise à disposition de l'hydroxyde de métal alcalino-terreux et de l'amine comprend également l'étape de mise à disposition de la diméthyléthanolamine avant la mise à disposition de l'hydroxyde de métal alcalino-terreux.
